# EUROPEAN PATENT APPLICATION

(11) **EP 4 414 003 A2**
(43) Date of publication of application: **14.08.2024**
(21) Application number: 24184725.0
(22) Date of filing: 02.05.2022
(51) Int. Cl.: A61M 1/00

(54) **NEGATIVE PRESSURE WOUND THERAPY DEVICE HAVING HELICAL ELEMENTS**

(30) Priority: 07.05.2021 US 202163186016 P; 04.03.2022 US 202263316453 P
(62) Divisional of application: 22721900.3
(71) Applicant: Ethicon, Inc., Raritan, NJ 08869 (US)
(72) Inventor: CICHOCKI, Frank, Raritan, 08869 (US); SLOSS, Scott, Raritan, 08869 (US); KILLION, John, Raritan, 08869 (US); MORTUS, Jared, Raritan, 08869 (US); COHN, William, Raritan, 08869 (US); SHARMA, Tushar, Raritan, 08869 (US); JACINTO, Gabriel, Raritan, 08869 (US)
(74) Representative: Carpmaels & Ransford LLP

(57) **Abstract**

A device is configured for use in negative pressure wound therapy (NPWT). The device includes a tube having a tube proximal end and a tube distal end and A coil having a plurality of windings extending from a coil proximal end to a coil distal end. The coil distal end is coupled to the tube proximal end in a manner that enables application of a negative pressure from within the coil through the coil to tissue surrounding the coil. The coil comprises a space between adjacent ones of the plurality of windings, the space having a predetermined dimension.

## Description

### PRIORITY CLAIM

The present disclosure claims priority to U.S. Provisional Patent Application Serial No. 63/186,016 filed May 7, 2021 and U.S. Provisional Patent Application Serial No. 63/316,453 filed March 4, 2022; the disclosures of which these applications are incorporated herewith by reference.

### TECHNICAL FIELD

The field of art to which the present disclosure pertains is Negative Pressure Wound Therapy (NPWT), specifically devices used for the approximation of tissues for improved wound healing and the prevention of wound complications.

### BACKGROUND

One of the issues associated with topical NPWT systems applied to surgical sites is that they often do not eliminate dead space or aid in the approximation of tissues beneath the skin surface.

Another issue is that topical NPWT often require the use of a dressing that must be precisely sealed to the skin to function properly.

Another issue is that conventional dressings are large, non-absorbable and difficult and painful to remove.

Another issue with topical NPWT systems is that the dressing may need to be changed multiple times over the course of the therapy to keep the surface of the surgical site clean and sanitary.

### SUMMARY

The present disclosure relates to a bioabsorbable device for the vacuum assisted approximation of tissues in a surgical site. This device, when used in conjunction with negative pressure, can facilitate intimate approximation of soft tissues deep within a surgical wound.

The present disclosure also relates to a device which is configured for use in negative pressure wound therapy (NPWT). The device includes a tube having a tube proximal end and a tube distal end and A coil having a plurality of windings extending from a coil proximal end to a coil distal end. The coil distal end is coupled to the tube proximal end in a manner that enables application of a negative pressure from within the coil through the coil to tissue surrounding the coil. The coil comprises a space between adjacent ones of the plurality of windings, the space having a predetermined dimension.

In addition, the present disclosure relates to a method for placing in a surgical site a device having a tube with a needle coupled to a distal end of the tube and a coil coupled to a proximal end of the tube. The method includes driving the needle from an interior of a wound through deep tissue and out through skin; cutting the needle free from the tube; connecting the device to a vacuum source using a fluid connector; applying a vacuum pressure to the device between about 50 mm Hg to about 130 mm Hg; and removing the tube from the coil to leave the coil in the wound at the end of the therapy.

Furthermore, the present disclosure relates to a method for approximating tissues within a living body. The method includes the steps of placing into surgical incision a device including a tube having a tube proximal end and a tube distal end and a coil having a plurality of windings extending from a coil proximal end to a coil distal end, wherein the coil distal end is coupled to the tube proximal end; and applying to a lumen of the coil a negative pressure so that the negative pressure is applied through the coil to tissue surrounding the coil to draw together tissue adjacent tissues of the incision.

### BRIEF DESCRIPTION OF THE DRAWINGS

The patent or application file contains at least one drawing executed in color. Copies of this patent with color drawings will be provided by the U.S. Patent and Trademark Office upon request and payment of necessary fee.
Fig. 1 shows a side view of a negative pressure wound therapy (NPWT) device according to embodiments of the present disclosure.
Fig. 2a shows a closeup side view of the junction between the tube and coil of the NPWT device of Fig. 1.
Fig. 2b shows a cross-sectional view of the junction between the tube and coil shown in Fig. 2a.
Fig. 3 shows a cross-sectional view of the coil of the NPWT device of Fig. 1.
Fig. 4a shows a top view of a negative pressure wound therapy (NPWT) device according to embodiments of the present disclosure.
Fig. 4b show a closeup view of the coil of the NPWT device of Fig. 4a.
Zshow a microscopic view of a gap between adjacent coils of the NPWT device of Fig. 4a.
Fig. 5 shows a cross-sectional view of a coil according to an embodiment in which the coil has a circular cross-section.
Fig. 6 shows a cross-sectional view of a coil according to an embodiment in which the coil has a square cross-section.
Fig. 7 shows a cross-sectional view of a coil according to an embodiment in which the coil has a first triangular cross-section.
Fig. 8 shows a cross-sectional view of a coil according to an embodiment in which the coil has a second triangular cross-section.
Fig. 9 shows a side view of a device according to a further embodiment.
Fig. 10 shows a partially cross-sectional side view of the device of Fig. 9.
Fig. 11 shows a partially cross-sectional view of a portion of the coil of the device of Fig. 9.
Fig. 12 shows a cross-sectional side view of a portion of the coil of the device of Fig. 9.

### DETAILED DESCRIPTION

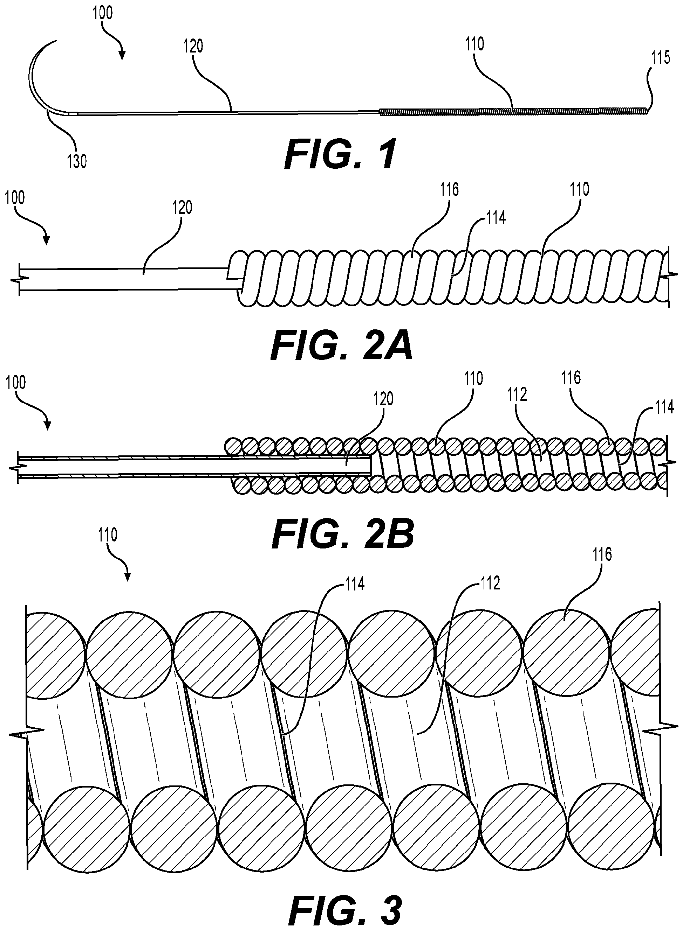

Fig. 1 is a side view of a negative pressure wound therapy (NPWT) device 100 according to embodiments of the present disclosure. The device 100 comprised of at least one helical component or coil 110 that is attached and in fluid communication with a continuous, unperforated tube 120. The continuous, unperforated tube 120 is then preferably attached to a suture needle 130 to facilitate easy and effective deployment of the device in a surgical site. The coil 110 and a tube 120 are preferably produced from the family of bioabsorbable polyesters commonly used in the field of suture manufacturing. A wide variety of the needles 130, or various size, curvature and needle point design may be attached to the tube 120 to accommodate the preference of the surgeon for installation at any tissue level within a surgical site. In addition, a proximal end of the coil 110 preferably includes a plug 115 to seal the proximal end of the coil 110 to prevent the introduction into the coil 110 of fluids or other debris that might clog the lumen of the coil 110.

Surgeries, pathologies and/or wounds can often separate layers of tissue or create deadspaces (voids created, e.g., via excision or other open areas) within tissues that it is desirable to close. As would be understood by those skilled in the art, the body generally reacts to such deadspaces within tissues by generating fluids that can have a deleterious effect on healing (e.g., permitting the spread of bacteria, etc.). Thus, eliminating deadspaces by bringing the tissues surrounding a deadspace together promotes healing and minimizes the deleterious effects of serous fluids mentioned above. Conventional treatments such as topically applied closed incision NPWT dressings have been effective in drawing together the edges of surface incisions but have been substantially ineffective in eliminating deadspaces deeper in tissue.

In addition, as would be understood by those skilled in the art, when drawing tissues together, it is generally desirable to spread the force applied to the tissue as evenly and over as wide a surface area as possible to eliminate stress concentrations on small areas of tissue. Those skilled in the art would understand that the elimination or minimization of such stress concentrations may also reduce blood perfusions that might result were such stress concentrations present. This is particularly helpful with tissue that is soft (e.g., fatty tissues) and which may react poorly to tissue approximating techniques such as suturing that apply such concentrated forces. The present embodiments apply vacuum pressures over a wide area to remove from deadspaces whatever may be found there (e.g., air, fluid, etc.) to draw together the tissue surrounding the deadspace.

As these devices are designed to operate in a wide range of environments surrounded by various tissues and in the presence of different fluids and/or gases, the construction of the devices is directed to maintaining open the channels through which the negative pressure is applied to the tissue while evacuating the contents of the deadspace. As would be understood by those skilled in the art, as negative pressure will continue to be applied until the user desires to end this treatment, fluids generated within the area treated may continue to be drawn out of the body via a placed device. However, to the extent that the deadspace is eliminated by drawing the opposing portions of tissue together, the generation of fluid will be substantially reduced in comparison to a situation where a drain is placed into a deadspace to remove fluids without drawing the surrounding tissues together into contact with one another.

Fig. 2a is a closeup side view of the junction between the tube 120 and the coil 110 of the device 100 of Fig. 1. Fig. 2b is a cross-sectional view of the junction between the tube 120 and the coil 110 shown in Fig. 2a. The tube 120 is at least partially disposed within the lumen 112 of the coil 110. In some embodiments, the tube 120 may be bonded to the coil 110 via thermal processing, solvent bonding, or use of bioabsorbable adhesives. In some embodiments, the tube 120 is alternatively press fit into the coil 110. In such an embodiment, the outside diameter of the tube 120 is slightly larger than the inside diameter of the coil 110. The depth of the press fitting and the design of the coil 110 may be adjusted to adjust the force required to detach the device 100 from the coil 110 at the end of therapy. A short segment of bioabsorbable filament may be press fit into the end of the coil 110 to seal the opposite end. Adhesive or thermal sealing methods may also be used.

Fig. 3 is a cross-sectional view of the coil 110 of the device 100 of Fig. 1. A gap 114 between filament loops 116 that comprise the coil 110 may be tailored to prevent blood clots, cells or cell clusters, and other wound detritus from clogging the fine tube (not shown) located between the coil 110 and the vacuum source (not shown). In some embodiments, the gap 114 may be less than 2 um which would allow for the filtering of single red blood cells which can be helpful in avoiding clotting within a device lumen that would inhibit the application of vacuum pressure to tissue as desired. In some embodiments, the gap 114 may alternatively be between about 2 µm and 80 µm to filter wound debris and while also preventing clogging of the tubing 120 and the lumen 112 of the coil 110.

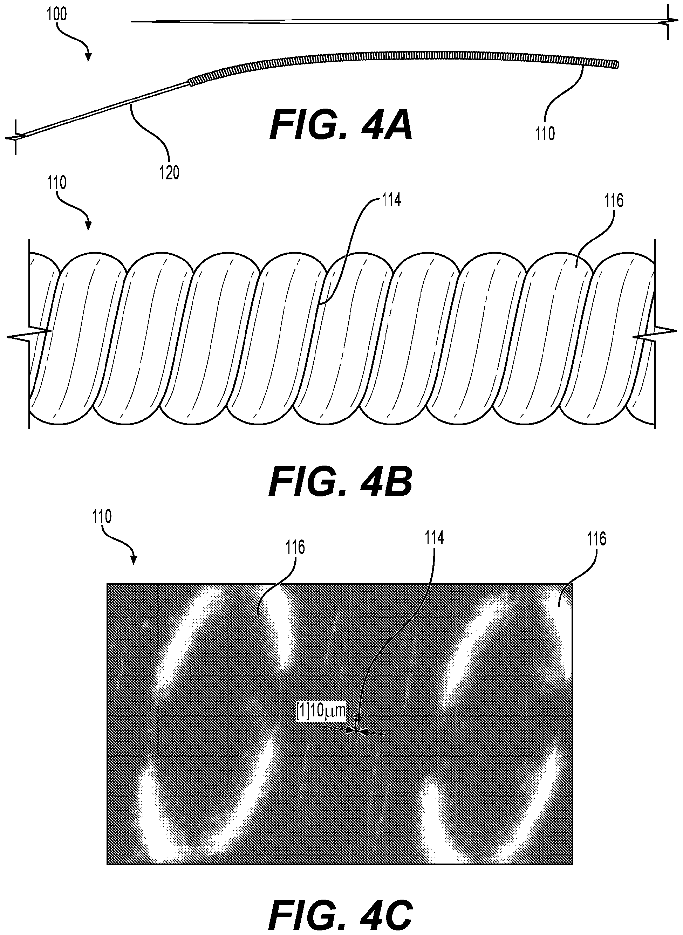

Fig. 4a is a top view of the device 100 according to embodiments of the present disclosure. Fig. 4b is a closeup view of the coil 110 of the device 100 of Fig. 4a. Fig. 4c is a microscopic view of the gap 114 between the adjacent loops 116 of the coil 110 of the device 100 of Fig. 4a. In some embodiments, the device 100 may be produced from a bioabsorbable polyester (e.g., Ethicon, Inc.'s MONOCRYL) tubing with a 0.021" inner diameter and a 0.032" outer diameter attached to the loops 116 produced from a size 2 (i.e., about 0.024 in) bioabsorbable polyester (e.g., Ethicon, Inc.'s polydioxanone) fiber.

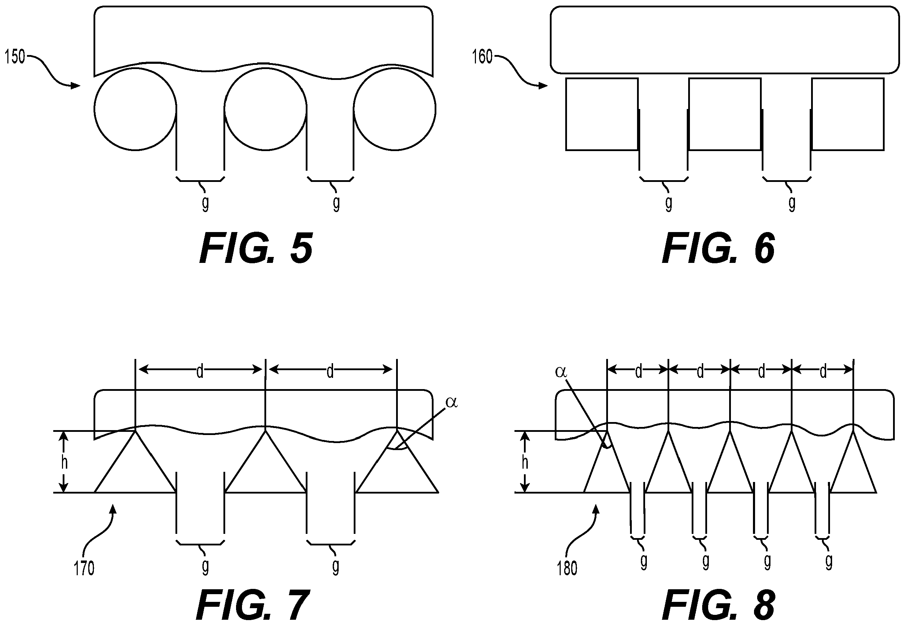

As shown in Figs. 5-8 and described in more detail below, the coil 110 according to various embodiments may have different cross-sectional shapes leading to different properties in regard to the application of negative pressure to surrounding tissue and the extent to which various types of surrounding tissues will interact with the coil 110. In some embodiments, the length of the coil 110 may be about 3.5" and the length of the tube 120 may be about 14". It should be noted, however, that a wide range of sizes and lengths are possible to address various surgical needs and incision sizes. It should also be noted that, instead of PDS coils and MONOCRYL tubes, the device (e.g., coil or microtube) may alternatively be made of other biocompatible and bioabsorbable materials such as, for example, PGA, PGA/PLA, other bioabsorbable polyesters, catgut, collagen, PVA, oxygen regenerated cellulose and the like.

In some embodiments, the method of placing of the device 100 is analogous to the method used for implantation of a Blake drain. The placement location would typically be between suture lines in a surgical wound requiring multi-layer closure. In some embodiments, a method of placing the device 100 includes driving the needle 130 from the interior of the wound through the deep tissue and out through the skin, cutting the needle 130 free from the tube 120, connecting the device 100 to a vacuum source using a fluid connector (e.g., a Toughy-Borst or other similar connector), applying a vacuum pressure between about 50 mm Hg to 130 mm Hg to the placed device 100, and removing the tube 120 from the coil 110 to leave the coil 110 at the wound site to bioabsorb at the end of the therapy. In some embodiments, the method may additionally include fixing the device 100 in place with adhesive strips and/or transparent adhesive patches to prevent accidental dislodgement.

In some embodiments, the vacuum pressure is continuously applied. In some embodiments, the vacuum pressure is alternatively cycled within a desired pressure range. The removal of the tube 120 from the coil 110 at the end of therapy may be performed through application of a modest force on the tube 120 to remove it from the coil 110. The coil 110 remains behind to bioabsorb. As an alternative, as would be understood by those skilled in the art, the device 100 may be detached from the patient by cutting the tube 120 at the skin surface and then applying a bandage. In addition, as would be understood by those skilled in the art, the loops 116 of the coil 110 of the disclosed embodiments are inherently strong permitting the delivery of significantly higher vacuum pressure levels (e.g., >>130 mm Hg) to tissue without collapsing the coil 110 and the coil 110 achieves greatly expanded "open area" (i.e., all the tissue surrounding the coil 110 is exposed to the vacuum pressure).

The device 100 disclosed herein is configured to enable vacuum-assisted tissue approximation at much finer dimensions and operates in a different manner on a wider area of tissue than the conventional silicone-based Blake drains. Both the design and the material of the device 100 are configured to enable the fine dimension. In an embodiment, the coil 110 may be an elongated spiral or helical section and the tube 120 may be a continuous unperforated microtube that is detachable from the elongate spiral or helical section. Moreover, the continuous wall element, when processed from a bioabsorbable polyester (e.g., Ethicon, Inc.'s MONOCRYL and/or polydioxanone, or PGA/PLA blends) with the correct draw ratio and anneal cycles to develop an optimal level of crystallinity is considerably stronger than the conventional silicone based devices used in the field today and is therefore much less susceptible to accidental breakage. In other words, if conventional drains were made to the scale of the device 100 described herein, these conventional drains could easily break during removal, leading to entrapment of a non-absorbable material in the wound site, which would necessitate an invasive re-operation for removal.

Additional benefits of the device 100 described herein when compared with conventional dressings/devices include: 1) reducing the risk of infection, since a decrease in the diameter of the device consequentially decreases the surface area at the intersection with the skin, and 2) large diameter device removal can be painful for the patient whereas finer diameter devices are typically associated with less pain during removal, resulting in an overall better experience for the patient. Furthermore, if the coil 110 of these embodiments were to be caught by a suture during would closure, this would have no impact on the procedure as the coil 110 would simply be absorbed into the body. In contrast, a re-operation would be required for the same situation with a conventional silicone drain. In addition, the flexibility of the coil 110 of these embodiments makes them beneficial in wounds to areas which are subject to significant movement and/or stresses (e.g., would in an around joints).

In some embodiments, the bioabsorbable polyesters used in the device 100 disclosed herein can be made to exhibit antimicrobial properties using, for example, a vapor infusion/coating with triclosan. The antimicrobial coating has been shown to exhibit clinical efficacy against many of the worst and most common bacteria associated with hospital acquired infection, including MRSA. The MONOCRYL and PDS sutures, which are part of Ethicon, Inc.'s PLUS family of offerings, are the same materials used in the NPWT prototypes described in this disclosure.

Most importantly, the device 100 described in this disclosure can offer several advantages over the state-of-the art NPWT surgical site dressings applied to the skin. Such NPWT surgical site wound dressings are generally comprised of a compressible, porous, absorbent material that is covered by an adhesive backed continuous airtight transparent dressing. This dressing is in then placed in communication with a tube and vacuum pump. One difficulty with such a dressing is associated with the fact that the dressing must be sealed well to the patients' skin in order to function properly. Any wrinkles, pin holes, or kinks in the dressing may result in a vacuum leak that renders the dressing ineffective.

Another issue with conventional NPWT dressings is, in some cases, the inability of the patient to shower. Yet another issue associated with conventional NPWT dressings is the need to change the dressing regularly, especially if the dressing becomes soiled or wet. Finally, conventional NPWT dressings applied topically to the surface of a surgical wound often are incapable of pulling a vacuum beyond the first layer of tissue at the skin level to which they are applied. As a result, the clinical benefit of superior tissue approximation is limited in large part to the skin level and tissues surrounding deep incisions are not approximated as desired.

As shown in Figs. 5-8, embodiments may employ coils having different cross-sections to achieve different results depending on the goals and/or the type of tissue within which the device will reside. For example, a coil 150 having the standard circular cross-section shown in Fig. 5 will have filtering and tissue supporting behavior as described above while a coil 160 having the square cross-section as shown in Fig. 6 (with a gap size g the same as the gap size g of the circular coil of Fig. 5) will have enhanced filtering capabilities. Those skilled in the art will understand that the filtering capability of the device will depend on the size of the gap g while the differing shape and spacing of the portions of the coils 150, 160 facing the surrounding tissue will impact the extent to which tissue is drawn into spaces between the adjacent turns of the coils 150, 160.

For example, a coil 170 as shown in Fig. 7 and a coil 180 as show in Fig. 8 each having triangular cross-sections, the gap g combined with the height and the vertex angle α of the triangles determine the distance d between adjacent peaks of the coil that contact the tissue and this will alter the extent to which tissue is drawn against or between the turns of the coils 170, 180 when negative pressure is applied. Thus, as would be understood by those skilled in the art, where tissue is more compliant it may be desirable to reduce the distance d by changing the vertex angle α and/or the height h to reduce d and, when tissue is more rigid d may be increased to enhance the exposure of surrounding tissue to the negative pressure.

As would be understood by those skilled in the art, the configuration of the coil 110 is selected to permit the application of vacuum force to a maximum surface area of tissue without pulling the tissue into the gap g and potentially sealing the device. Thus, for very soft tissue (e.g., adipose tissue) the coil 160 having a square cross-section (as shown in Fig. 6) may be desirable. Where tissue is less compliant (e.g., fibrous tissue) the use of the coil 170 with a triangular cross-section may be more desirable as the increased distance d enables the application of vacuum pressure over a wider surface area of tissue.

Conversely, the device 100 described herein does not require use of a dressing and can be used to more effectively approximate the deep tissue of a surgical site. As a result, the NPWT device described herein overcomes many of the limitations associated with certain conventional NPWT dressings as described above. An embodiment as described herein has been tested against a current NPWT system.

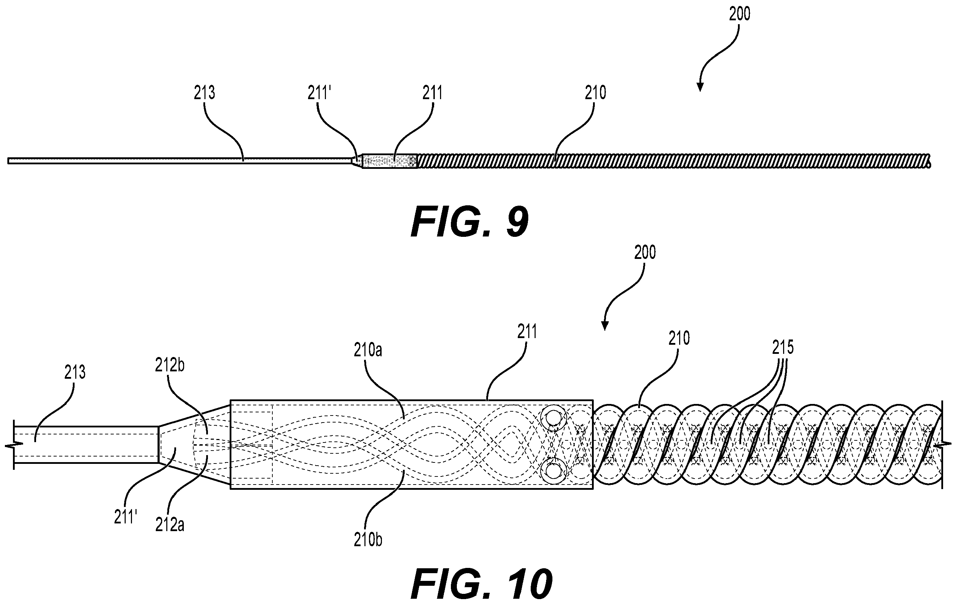

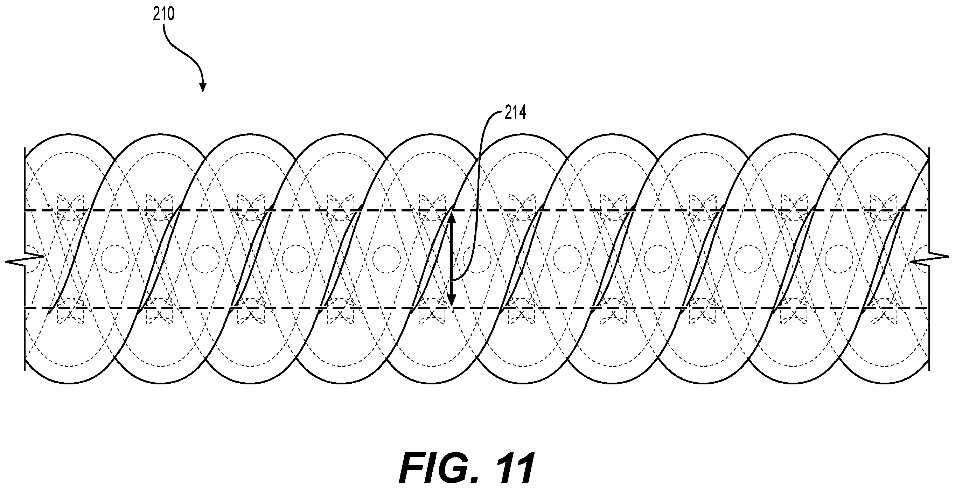

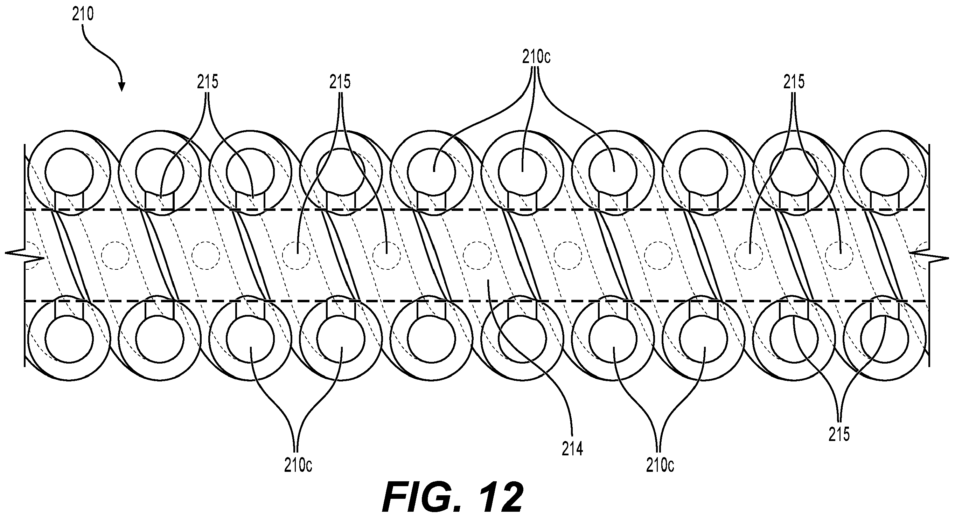

As shown in Figs. 9 - 12, a device 200 according to a further embodiment is constructed substantially similarly to the device 100 described above except for the differences described below. Specifically, the device 200 includes a coil 210 that is formed as a double helix in which a first hollow tube 210a is intertwined with a second hollow tube 210b so that the first and second tubes 210a and 210b each wind helically around a coil lumen 214 outlined generally by the dotted lines in Figs. 11 and 12. In addition, each of the tubes 210a and 210b defines a separate tube lumen 210c extending therein from a sealed proximal end (not shown) to a distal opening 212a (for tube 210a) and 212b (for tube 210b). Each of the tubes 210a and 210b includes a plurality of openings 215 facing into the coil lumen 214. In the embodiment pictured, each of the tubes 210a and 210b includes two openings 215 for each turn of each of the tubes 210a and 210b about the coil lumen 214. These openings 215 permit the introduction of suction into the coil lumen 214 at multiple locations along the length of the coil 210. This provides a level of redundancy allowing the continued application of suction to the coil lumen 214 even if a blockage develops that would prevent the introduction of suction via the coil lumen 214 itself to parts of the coil 210 located further from the source of suction than the blockage (e.g., where suction is applied via the distal end of the coil 210, into parts of the coil 210 more proximal than a blockage in the lumen 214). Those skilled in the art will understand that the number and spacing of the openings 215 may be varied as desired to obtain the desired distribution and level of suction along the coil lumen 214. As seen in Figs. 9 and 10, a distal portion of the coil 210 is received within a collar 211 with the distal openings 212a and 212b received in a tapered distal end 211' of the collar 211. The distal end 211' of the collar 211 is coupled to a proximal end of a tube 213 so that suction applied to the tube 213 is introduced into the distal openings 212a and 212b as well as into a space within the collar 211 that is open to the coil lumen 214. Thus, suction applied to the tube 213 is applied directly to the coil lumen 214 via the space within the collar 211 and is also applied to the coil lumen 214 via the openings 215 in the tubes 210a and 210b. As indicated above, this allows the device 200 to continue applying suction over a large portion of its length even if a blockage were to develop within the lumen 214. In fact, even if one or more of the openings 215 were to be blocked suction could still be applied via the coil lumen 214 or via any of the unblocked openings 215.

Those skilled in the art will understand that a device similar to the device 100 could be formed including a single coil formed of a hollow tube with openings similar to the openings 215 of the device 200 providing a similar redundancy to a single coil apparatus. In this case, a collar such as the collar 211 may be applied if desired. Alternatively, suction may be applied via one source to the coil lumen itself while a second source of suction is applied to an open end of the single coiled hollow tube. Additionally, those skilled in the art will also understand that although two tubes 210a and 210b have been described and illustrated, the coil 210 may alternatively be formed of more than two tubes that are helically wound.

Although the present disclosure has been shown and described with respect to detailed embodiments thereof, it will be understood by those skilled in the art that various changes in form and detail thereof may be made without departing from the spirit and scope of the claimed invention. Furthermore, it should be understood by those skilled in the art that any of the features of one embodiment may be combined with the features of any other embodiment in any manner that is not inconsistent with or expressly disclaimed by the disclosure.

The following is a non-exhaustive list of numbered embodiments which may be claimed:
1. A device configured for use in negative pressure wound therapy (NPWT), comprising:
   a tube having a tube proximal end and a tube distal end; and
   a coil having a plurality of windings extending from a coil proximal end to a coil distal end, wherein the coil distal end is coupled to the tube proximal end in a manner that enables application of a negative pressure from within the coil through the coil to tissue surrounding the coil, and wherein the coil comprises a space between adjacent ones of the plurality of windings, the space having a predetermined dimension.
2. The device of embodiment 1, wherein the coil is a helical coil detachably coupled to the tube so that the coil may be removed from the tube.
3. The device of embodiment 1, wherein the tube proximal end is sealed to prevent the introduction therein of materials.
4. The device of embodiment 1, further comprising a suture needle coupled to the tube distal end.
5. The device of embodiment 1, wherein the tube proximal end is press fit into the coil distal end.
6. The device of embodiment 5, wherein a connection between the coil and the tube is configured to permit, after placement of the device at a desired location within tissue, the tube to be pulled free from the coil when a force of at least a predefined release amount is applied between the coil and the tube.
7. The device of embodiment 1, wherein the coil is formed of a bioabsorbable material.
8. The device of embodiment 1, wherein the predetermined dimension of the space is between 2µm - 80 µm.
9. The device of embodiment 1, wherein the predetermined dimension of the space is between 40 µm - 60 µm.
10. The device of embodiment 1, wherein the coil includes an antimicrobial.
11. The device of embodiment 10, wherein the antimicrobial is within the polymer or formed as a coating on a surface of the polymer.
12. The device of embodiment 1, wherein a cross-section of the coil is one of round, square, or triangular.
13. The device of embodiment 12, wherein, when the coil has a triangular cross-section, a vertex angle and height of the coil are selected to achieve a desired distance between adjacent turns of the coil at radially outermost points of the coil.
14. The device of embodiment 1, wherein the coil is formed of a first hollow tube wrapped about a coil lumen and wherein the first hollow tube includes a plurality of first openings open from a first tube lumen within the first hollow tube to the coil lumen.
15. The device of embodiment 14, wherein the coil includes a second hollow tube intertwined with the first hollow tube to wrap around the coil lumen in a double helix and wherein the second hollow tube includes a plurality of second openings open from a second tube lumen within the second hollow tube to the coil lumen.
16. The device of embodiment 15, further including a collar encasing a distal portion of the coil wherein the coil lumen and open distal ends of the first and second hollow tubes are open to a distal opening of the coil so that suction applied to the collar is applied to the coil lumen and to the first and second openings.
17. A method for placing a device in a wound, the device having a tube with a needle coupled to a distal end of the tube and a coil coupled to a proximal end of the tube, the method comprising:
   driving the needle from an interior of the wound through deep tissue and out through skin;
   cutting the needle free from the tube;
   connecting the device to a vacuum source using a fluid connector;
   applying a vacuum pressure to the device between about 50 mm Hg to about 130 mm Hg; and
   removing the tube from the coil to leave the coil in the wound at the end of the therapy.
18. The method of embodiment 17, further comprising:
   fixing the device in place with adhesive strips to prevent accidental dislodgement of the device.
19. The method of embodiment 17, wherein the vacuum pressure is continuously applied.
20. The method of embodiment 17, wherein the vacuum pressure is cycled within a desired pressure range.
21. A method for approximating tissues within a living body, comprising the steps of:
   placing into a deadspace formed within tissue a device including a tube having a tube proximal end and a tube distal end and a coil having a plurality of windings extending from a coil proximal end to a coil distal end, wherein the coil distal end is coupled to the tube proximal end; and
   applying to a lumen of the coil a negative pressure so that the negative pressure is applied through the coil to tissue surrounding the coil to draw together tissue surrounding the deadspace.
22. The method of embodiment 21, wherein the coil is configured so that a spacing between adjacent ones of the plurality of turns of the coil prevents entry into the coil of red blood cells.
23. The method of embodiment 21, wherein a cross-sectional shape and size of the material of the coil is selected based on properties of a type of tissue surrounding the deadspace.

## Claims

1. A device configured for use in negative pressure wound therapy (NPWT), comprising:
a tube having a tube proximal end and a tube distal end; and
a coil having a plurality of windings extending from a coil proximal end to a coil distal end, wherein the coil distal end is coupled to the tube proximal end in a manner that enables application of a negative pressure from within the coil through the coil to tissue surrounding the coil, and wherein the coil comprises a space between adjacent ones of the plurality of windings, the space having a predetermined dimension.

2. The device of claim 1, wherein the coil is a helical coil detachably coupled to the tube so that the coil may be removed from the tube.

3. The device of claim 1, wherein the tube proximal end is sealed to prevent the introduction therein of materials.

4. The device of claim 1, further comprising a suture needle coupled to the tube distal end.

5. The device of claim 1, wherein the tube proximal end is press fit into the coil distal end.

6. The device of claim 5, wherein a connection between the coil and the tube is configured to permit, after placement of the device at a desired location within tissue, the tube to be pulled free from the coil when a force of at least a predefined release amount is applied between the coil and the tube.

7. The device of claim 1, wherein the coil is formed of a bioabsorbable material.

8. The device of claim 1, wherein the predetermined dimension of the space is between 2µm - 80 µm.

9. The device of claim 1, wherein the predetermined dimension of the space is between 40 µm - 60 µm.

10. The device of claim 1, wherein the coil includes an antimicrobial.

11. The device of claim 10, wherein the antimicrobial is within the polymer or formed as a coating on a surface of the polymer.

12. The device of claim 1, wherein a cross-section of the coil is one of round, square, or triangular.

13. The device of claim 12, wherein, when the coil has a triangular cross-section, a vertex angle and height of the coil are selected to achieve a desired distance between adjacent turns of the coil at radially outermost points of the coil.
